# EUROPEAN PATENT SPECIFICATION

(11) **EP 4 526 683 B1**
(45) Date of publication and mention of the grant of the patent: **03.12.2025**
(21) Application number: 23726545.9
(22) Date of filing: 19.05.2023
(51) Int. Cl.: G01N 33/574

(54) **METHOD FOR IDENTIFYING CANCER PATIENTS THAT BENEFIT FROM ANTI-CLEVER-1 TREATMENT**
VERFAHREN ZUR IDENTIFIZIERUNG VON KREBSPATIENTEN, DIE VON EINER ANTI-CLEVER-1-BEHANDLUNG PROFITIEREN
MÉTHODE D'IDENTIFICATION DE PATIENTS ATTEINTS D'UN CANCER QUI BÉNÉFICIENT D'UN TRAITEMENT ANTI-CLEVER-1

(30) Priority: 20.05.2022 FI 20225445; 26.10.2022 FI 20225962
(43) Date of publication of application: 26.03.2025
(73) Proprietor: Faron Pharmaceuticals OY, 20520 Turku (FI)
(72) Inventor: JALKANEN, Juho, 23120 Mietoinen (FI); BJÖRKMAN, Mari, 20720 Turku (FI); HOLLMÉN, Maija-Leena, 20760 Piispanristi (FI)
(74) Representative: Berggren Oy
(86) International application number: PCT/FI2023/050280
(87) International publication number: WO 2023/222953

(56) References cited:
- WO-A1-2010/122217
- WO-A1-2021/058866
- WO-A1-2021/094652
- ANONYMOUS: "Product Datasheet Stabilin-1 Antibody (4G9) H00023166-M05", 22 August 2021 (2021-08-22), pages 1 - 4, XP093059143, Retrieved from the Internet <URL:https://www.novusbio.com/PDFs/H00023166-M05.pdf> [retrieved on 20230630]
- VIRTAKOIVU REETTA ET AL: "Systemic Blockade of Clever-1 Elicits Lymphocyte Activation Alongside Checkpoint Molecule Downregulation in Patients with Solid Tumors: Results from a Phase I/II Clinical Trial", CLINICAL CANCER RESEARCH, vol. 27, no. 15, 1 August 2021 (2021-08-01), US, pages 4205 - 4220, XP055837789, ISSN: 1078-0432, DOI: 10.1158/1078-0432.CCR-20-4862

## Description

### Field of the invention

The present invention relates to a pre-treatment identification of cancer patients that will respond to a treatment comprising an administration of an agent capable of binding to CLEVER-1, using immunohistochemistry staining of a tumor biopsy prior to the treatment.

### Background of the invention

Currently, immune checkpoint inhibitors targeting CTLA-4 and the PD-1/PD-L1 axis are approved for clinical use, and while highly efficacious in about 10-20 % of patients, the majority of cancer patients do not respond to checkpoint inhibitors and other novel immunotherapies. A key to success in immunotherapy is to identify the patients that will respond [1]. Patients responding favorably to checkpoint inhibition usually have a pre-existing antitumor immune response, which is characterized by high density of IFNγ-producing CD8⁺ T cells, expression of PD-L1 in tumor-infiltrating immune cells, and high mutational load. PD-L1 expression in a pre-treatment biopsy is often used to pick patients for anti-PD-1 or anti-PD-L1 treatment.

Anti-CLEVER-1 is a novel immunotherapy targeting tumor associated macrophages, which has shown promising single agent efficacy in a first-in-human (FiH) clinical trial named MATINS (clinicaltrials.gov NCT03733990: A Study to Evaluate Safety, Tolerability and Preliminary Efficacy of FP-1305 in Cancer Patients (MATINS)) [2].

Innate immune cells such as macrophages, can dampen T cell activation and contribute to tumor progression despite high mutational load. The macrophages that contribute to tumor-related immunosuppression and provide tumor growth supporting signals may be highly eligible candidates for targeted therapies, since these cells are abundantly present in various tumors, they are very plastic and can be converted into pro-inflammatory macrophages supporting T cell activation and tumor killing [3, 4]. CLEVER-1 (also known as STABILIN-1) is a multifunctional molecule conferring scavenging ability on a subset of anti-inflammatory macrophages [5, 6]. However, there is still a need to identify the patients that will respond to anti-CLEVER-1 therapy and to find methods for the pre-treatment identification and patient selection for reducing or even eliminating un-necessary treatments.

WO 2010/122217 A1 discloses a method for pre-treatment identification of cancer patients that would respond to anti-CLEVER-1 therapy, based on the detection of the presence of CLEVER-1 expressing cells in a tumor sample obtained from a cancer patient by immuno-histochemistry staining.

### Summary of the Invention

It is anticipated that cancer patients having CLEVER-1 positive macrophages would benefit most from the anti-CLEVER-1 treatment, but now an accurate method has been found for identifying cancer patients that respond to a treatment comprising an administration of an agent capable of binding to CLEVER-1, preferably anti-CLEVER-1 antibody, more preferably anti-CLEVER-1 antibody bexmarilimab. Especially it has been found an accurate method for pre-treatment identification of cancer patients, which method provides accurate results by machine reading from the stained samples using a specific staining antibody. The present invention is based on the staining by a specific antibody which is now found to be suitable for machine reading and does not cause background color which may disturb machine reading of the stained samples. In addition, it has been found that a specific composition of CLEVER-1 positive cells in the tumor content/biopsy is necessary to identify patients that benefit from anti-CLEVER-1 therapy.

It has been observed that cancer patients having at least 1 % of CLEVER-1 expressing intra-tumoral cells from the total amount of the viable intra-tumoral cells in a pre-treatment tumor sample, such as tumor biopsy according to the present invention are the most responsive to therapy comprising an administration of an agent capable of binding to CLEVER-1, preferably therapy comprising an administration of anti-CLEVER-1 antibody, more preferably therapy comprising an administration of anti-CLEVER-1 antibody bexmarilimab. Hence, it has been found that a threshold value for selection of a cancer patient to be treated by anti-CLEVER-1 agent, such as anti-CLEVER-1 antibody therapy is at least 1 %, calculated by the percentage of intra-tumoral CLEVER-1 expressing cells from total amount of viable intra-tumoral cells. In the method according to the present invention, CLEVER-1 expression is determined by immunohistochemistry (IHC) staining of a tumor sample prior to the beginning of anti-CLEVER-1 therapy. The present invention discloses the detailed method for the evaluation of the CLEVER-1 expression and provides a tool for patient selection.

An object of the present invention is to provide an accurate method for a pre-treatment identification and patient selection of cancer patients that respond to an anti-CLEVER-1 therapy using immunohistochemistry staining of a tumor biopsy prior to the beginning of the anti-CLEVER-1 therapy, and thus reduce or even eliminate un-necessary treatments. More particularly, the present invention provides a method for a pre-treatment identification and patient selection of cancer patient that respond to an anti-CLEVER-1 treatment comprising an administration of anti-CLEVER-1 antibody, preferably treatment comprising an administration of anti-CLEVER-1 antibody bexmarilimab. By the method according to the invention, the cancer patient to be treated by anti-CLEVER-1 therapy can be chosen and the threshold value of CLEVER-1 intra-tumoral expressing cells (percentage of CLEVER-1 expressing intra-tumoral cells from the total amount of the viable intra-tumoral cells) is about 1 %, based on immunohistochemistry staining of a tumor biopsy prior to the beginning of anti-CLEVER-1 therapy. When the tumor sample, such as tumor biopsy obtained from a cancer patient comprises at least 1 % of CLEVER-1 expressing intra-tumoral cells from the total amount of the viable intra-tumoral cells, it is an indication that the cancer patient is responsive to the anti-CLEVER-1 treatment, preferably anti-CLEVER-1 antibody therapy, more preferably anti-CLEVER-1 antibody bexmarilimab therapy.

An object of the present invention is also to provide method for treating a specific patient group with an improved treatment response, which patients are selected by a method according to the present invention.

In order to achieve among others, the objects presented above, the invention is characterized by what is presented in the enclosed independent claims. Some preferred embodiments of the invention will be described in the other claims. The embodiments and advantages mentioned in this disclosure are applicable, both to the method and to the uses according to the invention, even though it is not always specifically mentioned.

The present invention provides *in vitro* method for pre-treatment identification of cancer patients, in which method a tumor sample obtained from a cancer patient is studied. A typical method according to the present invention for pre-treatment identification of cancer patients that will respond to anti-CLEVER-1 therapy comprising an administration of an agent capable of binding to Common Lymphatic Endothelial and Vascular Endothelial Receptor-1 (CLEVER-1) in a patient, the method comprises
- detecting the presence of CLEVER-1 expressing cells in a tumor sample obtained from a cancer patient by immunohistochemistry staining using a mouse monoclonal IgG2a kappa STAB-1 antibody (clone 4G9), and
- calculating a percentage of intra-tumoral CLEVER-1 expressing cells from the total amount of viable intra-tumoral cells present in the stained tumor sample,
wherein the stained tumor sample which comprises at least 1 % of CLEVER-1 expressing intra-tumoral cells from the total amount of the viable intra-tumoral cells is an indication that the cancer patient is responsive to the anti-CLEVER-1 therapy.

The present invention also concerns a method for treating a cancer patient, which method comprises an administration of a therapeutically effective amount of an agent capable of binding to CLEVER-1, preferably anti-CLEVER-1 antibody, more preferably anti-CLEVER-1 antibody bexmarilimab, and in which method the patient is diagnosed with a tumour which shows at least 1% of CLEVER-1 expressing intra-tumoral cells from the total amount of the viable intra-tumoral cells by the immunohistochemistry staining according to the present invention. More particularly, the present invention relates to an agent capable of binding to CLEVER-1, preferably anti-CLEVER-1 antibody, more preferably anti-CLEVER-1 antibody bexmarilimab for use in a treatment of cancer in a patient having diagnosed with a tumour which comprises at least 1 % of intra-tumoral CLEVER-1 expressing cells from the total amount of the viable intra-tumoral cells in a sample obtained from the tumor. According to the present invention an agent capable of binding to CLEVER-1, preferably anti-CLEVER-1 antibody, more preferably anti-CLEVER-1 antibody bexmarilimab is used in treating cancer by reducing malignant tumour growth in a patient and/or by inhibiting metastasis formation.

The present invention provides a method for treating cancer patient, which method comprises at least the following steps of
- obtaining a tumor sample from a cancer patient,
- detecting the presence of CLEVER-1 expressing cells in the tumor sample obtained from the cancer patient by immunohistochemistry staining using a mouse monoclonal IgG2a kappa STAB-1 antibody (clone 4G9),
- calculating a percentage of intra-tumoral CLEVER-1 expressing cells from the total amount of viable intra-tumoral cells present in the stained tumor sample, and
- beginning an administration of an agent capable of binding to CLEVER-1, preferably an anti-CLEVER-1 antibody, more preferably anti-CLEVER-1 antibody bexmarilimab in a patient, when the stained tumor sample comprises at least 1 % of CLEVER-1 expressing intra-tumoral cells from the total amount of the viable intra-tumoral cells.

Further, the present invention relates to use of a kit comprising at least a mouse monoclonal IgG2a kappa STAB-1 antibody (clone 4G9) and reagents for staining a tumor sample, for carrying out a method according to the present invention for pre-treatment identification of cancer patient.

The present invention is based on the findings that the selection of patients that benefit from anti-CLEVER-1 therapy, preferably anti-CLEVER-1 antibody therapy, more preferably anti-CLEVER-1 antibody bexmarilimab therapy is more accurate and efficient when using the disclosed immunohistochemical staining method and calculating CLEVER-1 expression in the viable intra-tumoral cells. Based on the results, intra-tumoral CLEVER-1 expression levels alone were statistically significant to identify patients that benefit from anti-CLEVER-1 therapy from those that do not.

Based on the pre-treatment identification method according to the present invention, an agent capable of binding CLEVER-1, preferably anti-CLEVER-1 antibody, and more preferably anti-CLEVER-1 antibody bexmarilimab can be efficiently used in the treatment of cancer in a patient.

### Brief Description of the drawings

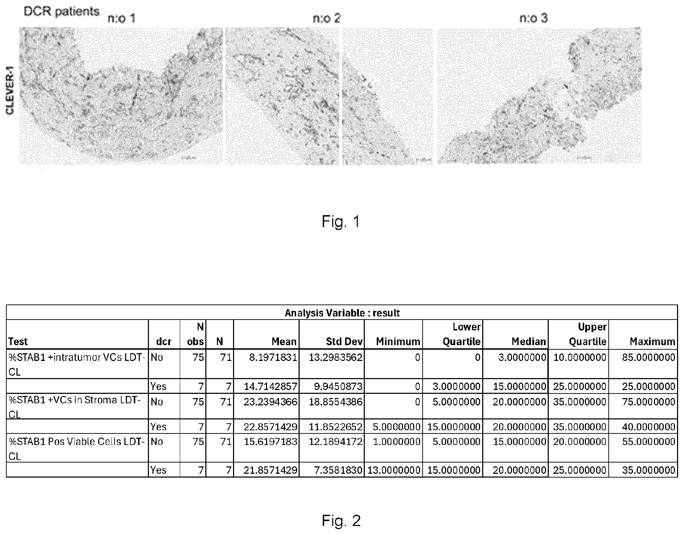

**Figure 1****.** Pre-treatment tumor biopsies from a first-in-human anti-CLEVER-1 trial named MATINS (clinicaltrials.gov NCT 03733990) from patients that a clinical benefit (extension of survival) from anti-CLEVER-1 treatment has observed. CLEVER-1 staining: black or dark grey (brown colour in actual) identifying CLEVER-1 positive cells. Patients that see a clinical benefit from anti-CLEVER-1 antibody treatment have a substantial amount of CLEVER-1 positive intra-tumoral cells.

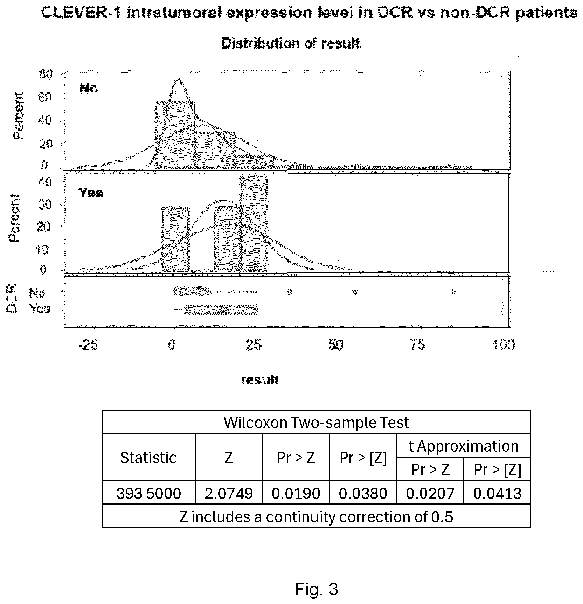

**Figures 2** **and** **3****.** The percentage of viable cells (VCs) that are positive for CLEVER-1 from the amount of total cells in a biopsy obtained from patient prior to anti-CLEVER-1 therapy in the MATINS Trial, and their association with clinical benefit (DCR = disease control rate). The percentage of CLEVER-1 positive intra-tumoral, but not stromal or all cells from biopsy associate with DCR (p = 0.038 in Fig. 3).

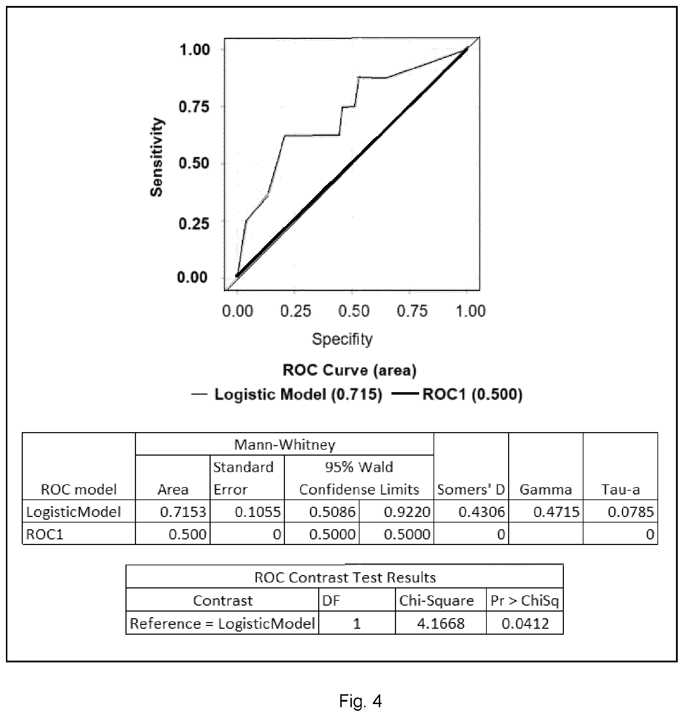

**Figure 4****.** Receiver operating characteristics (ROC) curve from data obtained on CLEVER-1 staining from pre-treatment tumor biopsies from the first-in-human anti-CLEVER-1 trial named MATINS investigating bexmarilimab (anti-CLEVER-1 mAb) for the treatment of advanced solid tumors (clinicaltrials.gov NCT 03733990). ROC curve is a plot of true positive rate (sensitivity) vs false positive rate (100-specificity) across all observed values of the diagnostic test and the Area Under Curve (AUC) of ROC curve describes the diagnostic tests' ability to detect cases from non-cases (1 = perfect separation, 0.5 = no separation based on diagnostic test). In the current ROC analysis using pre-treatment intra-tumoral staining result, the AUC value was 0.72 (95% confidence interval from 0.51 to 0.92).

### Detailed description of the invention

CLEVER-1 is a protein disclosed in the patent publication WO 03/057130, Common Lymphatic Endothelial and Vascular Endothelial Receptor-1. It is a binding protein that mediates adhesion of lymphocytes (and malignant tumor cells) to endothelium in both the systemic vasculature and in the lymphatics. CLEVER-1 (also known as STABILIN-1) is a multifunctional molecule conferring scavenging ability on a subset of anti-inflammatory macrophages [5, 6]. In these cells, it is involved in receptor-mediated endocytosis and recycling, intracellular sorting, and transcytosis of altered and normal self-components. More recently, it has been found that the progression of melanoma tumor growth and metastasis is attenuated in Stab1^{-/-} (Clever-1 knock out) mice, and in mice treated with anti-Clever-1 therapy [7]. More recently, similar results have now been achieved also in melanoma patients, as well as other cancers [2] using a novel humanized anti-CLEVER-1 antibody (FP-1305) named bexmarilimab disclosed in patent publication WO2017/182705. By blocking the interaction of CLEVER-1 and its lymphocyte substrate, it is possible to simultaneously control lymphocyte recirculation and lymphocyte migration, and related conditions such as inflammation, at the site of lymphocyte influx into, and efflux from, the tissues.

The terms "an agent capable of binding to CLEVER-1" and "anti-CLEVER-1 agent", refer to agents including antibodies and fragment(s) thereof, peptides or the like, which are capable of binding to CLEVER-1 for blocking the interaction of CLEVER-1 and malignant tumor cells. An agent capable of binding to CLEVER-1 may also be any other inhibitor, such as a small molecule inhibitor or macromolecule having an adequate affinity to bind to CLEVER-1 receptor and to inhibit the protein activity. The term "an antibody or fragment(s) thereof" is used in the broadest sense to cover an antibody or fragment(s) thereof which are capable to bind CLEVER-1 molecule in an individual. Especially, it shall be understood to include chimeric, humanized or primatized antibodies, as well as antibody fragment(s) and single chain antibodies (e.g. Fab, Fv), so long they exhibit the desired biological activities. Particular useful agents are anti-CLEVER-1 antibodies and fragment(s) thereof. Therefore, according to an embodiment of the present invention the agent capable of binding to CLEVER -1 is selected from the group consisting of an antibody or fragment(s) thereof, peptide(s), macromolecule and any combination thereof. According to the present invention "anti-CLEVER-1 treatment" or "anti-CLEVER-1 therapy" refers to the treatment comprising administration of at least one agent capable of binding CLEVER-1, preferably anti-CLEVER-1 antibody, more preferably anti-CLEVER-1 antibody bexmarilimab.

According to an embodiment of the invention, an agent capable of binding to CLEVER-1 comprises anti-CLEVER-1 antibody. Typically, an anti-CLEVER-1 antibody is a therapeutic humanized anti-CLEVER-1 antibody. According to an embodiment of the present invention an anti-CLEVER-1 antibody is a humanized monoclonal anti-CLEVER-1 antibody, previously presented in the patent publication WO2017/182705.

In an embodiment of the present invention, an anti-CLEVER-1 antibody is a humanized monoclonal immunoglobulin G4 kappa antibody bexmarilimab (International Nonproprietary Name (INN)) as disclosed in WHO Drug Information, Vol. 34, No. 3 (2020), pages 699-700), or bexmarilimab variant or the antibody in a bexmarilimab biosimilar. As used herein, "bexmarilimab" means the humanized monoclonal IgG4 antibody with the structure described in WHO Drug Information, Vol. 34, No. 3 (2020).

A bexmarilimab biosimilar means a biological product which is approved by a regulatory agency in any country for marketing as a bexmarilimab biosimilar. In an embodiment, a bexmarilimab biosimilar comprises a bexmarilimab variant as the drug substance. In an embodiment, a bexmarilimab biosimilar has substantially the same amino acid sequence of heavy and light chains as bexmarilimab. As used herein, a "bexmarilimab variant" means an antibody which comprises sequences of heavy chain and light chain that are identical to those in bexmarilimab, except for having one or more conservative amino acid substitutions at positions that are located outside of the light chain CDRs and/or one or more conservative amino acid substitutions that are located outside of the heavy chain CDRs, e.g. the variant positions are located in the framework regions or the constant region. In other words, bexmarilimab and a bexmarilimab variant comprise identical CDR sequences, but differ from each other due to having a conservative amino acid substitution at other positions in their full-length light and heavy chain sequences. A bexmarilimab variant is substantially the same as bexmarilimab with respect to binding affinity to CLEVER-1.

According to an embodiment of the present invention, a cell line producing the therapeutic anti-CLEVER-1 antibody bexmarilimab (FP-1305) has been deposited on 27 May 2020 under the terms of the Budapest Treaty on the International Recognition of the Deposit of Micro-organisms for the Purposes of Patent Procedure with the DSMZ-German Collection of Microorganisms and Cell Cultures GmbH, Inhoffenstrasse 7B, D-38124 Braunschweig, Germany, and has the accession number DSM ACC3361. The present invention is not to be limited in scope by the culture deposited, since the deposited embodiment is intended as a single illustration of one aspect of the invention and any culture that is functionally equivalent is within the scope of this invention. The deposit of material herein does not constitute an admission that the written description herein contained is inadequate to enable the practice of any aspect of the invention, including the best mode thereof, nor is it to be construed as limiting the scope of the claims to the specific illustration that it represents.

In the present invention, it has been found an efficient and accurate method for selecting cancer patients that respond to anti-CLEVER-1 treatment or therapy comprising an administration of an agent capable of binding to CLEVER-1, preferably anti-CLEVER-1 antibody, more preferably anti-CLEVER-1 antibody bexmarilimab in a patient. In a method according to the present invention a tumor sample is obtained from a cancer patient prior to the beginning of anti-CLEVER-1 treatment comprising an administration of an agent capable of binding to CLEVER-1, preferably anti-CLEVER-1 antibody, more preferably anti-CLEVER-1 antibody bexmarilimab in a patient, and based on the tumor sample it is evaluated the amount of the intra-tumoral cells expressing CLEVER-1 for making the pre-treatment identification and patient selection of cancer patients that respond to the anti-CLEVER-1 treatment.

According to an embodiment of the present invention, a tumor sample is a tumor biopsy sample. There are many different types of biopsy procedures for obtaining a tumor sample. A tumor sample may also be obtained using any other suitable method. A biopsy may be the removal of a small piece of tissue or a sample of cells. A tumor sample may comprise cells and/or a small piece of tissue. In an embodiment of the invention, a tumor sample is a tumor tissue sample.

According to an embodiment of the present invention a tumor tissue sample may be stained and examined for CLEVER-1 expressing cells as a fresh sample. According to other embodiment of the present invention, a tumor sample may be preserved by fixing and embedding in formalin, paraffin or the like. Hence, a tumor sample used in the method according to the present invention may be formalin or paraffin fixed sample or frozen sample. In a preferred embodiment, the tissue sample is fixed with formalin. In some embodiments, the fixed tissue sample is embedded in paraffin to prepare a formalin-fixed and paraffin-embedded (FFPE) tissue sample. Cross-linking fixatives, such as formalin, are used to prepare samples for immunohistochemistry staining because they preserve the structural integrity of the tumor tissue, which helps maintain tissue architecture. A tissue sample may be fixed by conventional methodology known in the art, with the length of fixation depending on the size of the tissue sample and the fixative used. Another option is to use frozen samples, wherein the tumor sample is e.g. cryopreserved.

In a method according to the present invention, CLEVER-1 expression is detected or determined in the cells of the tumor sample by immunohistochemistry staining using the specific antibody. The immunohistochemistry staining is carried out by known protocols in the art or provided by the producer of the staining antibody. Two general methods of immunohistochemistry staining may be used: direct and indirect assays. The immunohistochemistry staining may be performed using automated systems. In a method according to the invention, CLEVER-1 expressing cells is detected by staining the cells of the tumor sample with a mouse monoclonal IgG2a kappa STAB-1 antibody (clone 4G9). Further, a method according to the present invention for pre-treatment identification of cancer patients comprises calculating a percentage of intra-tumoral CLEVER-1 expressing cells from the total amount of viable intra-tumoral cells present in the stained tumor sample, wherein a tumor sample which comprises at least 1 % of CLEVER-1 expressing intra-tumoral cells from the total amount of viable intra-tumoral cells, is an indication that the cancer patient is responsive to the anti-CLEVER-1 therapy.

In a method according to the present invention, CLEVER-1 expression is calculated only CLEVER-1 expressing intra-tumoral cells divided by the total number of viable intra-tumoral cells in the sample, multiplied by 100. According to an embodiment of the present invention intra-tumoral cells comprises macrophages and/or tumor endothelial cells.

In the pre-treatment screening method according to the present invention, it has been observed that CLEVER-1 staining by a highly specific anti-CLEVER-1 antibody, more particularly a mouse monoclonal IgG2a kappa STAB-1 antibody (clone 4G9) is needed for proper staining of CLEVER-1 positive intra-tumoral cells. A monoclonal IgG2a kappa STAB-1 antibody (clone 4G9) is produced in mouse clone 4G9. A mouse monoclonal IgG2a kappa STAB-1 antibody (clone 4G9) is raised against amino acids 1804-1902 of STABILIN-1 (CLEVER-1) of human origin (SEQ ID NO: 1). In an exemplary embodiment, anti-CLEVER-1 antibody used in the method according to the present invention is STAB1 monoclonal antibody (M05), clone 4G9 from Abnova (Taiwan) Corporation (product details: http://www.abnova.com/products/products_detail.asp?catalog id=H0002316 6-M05). The immunohistochemistry staining can be carried out based on the protocol provided by the producer of the antibody. The amount of CLEVER-1 expressing cells in a tumor sample may be calculated by manually by pathologist using microscopy, but for achieving more accurate results, samples are stained and machine read by automated systems. Hence, the staining need to be done by antibody which is suitable for machine reading and does not cause background color which may disturb machine reading of the stained samples. In the present invention, it has been found that a mouse monoclonal IgG2a kappa STAB-1 antibody (clone 4G9) provides accurate staining without interfering background color, and therefore the proper staining for pre-treatment evaluation is achieved by a mouse monoclonal IgG2a kappa STAB-1 antibody (clone 4G9) and the calculating of the CLEVER-1 expressing cells can be done by machine reading accurately.

In a method according to the present invention, an obtained tumor sample is stained in an immunohistochemical (IHC) assay with an antibody that specifically binds to CLEVER-1 protein, wherein the antibody is a mouse monoclonal IgG2a kappa STAB-1 antibody (clone 4G9).

The first human data using a humanized anti-CLEVER-1 antibody (bexmarilimab) from the MATINS trial (clinicaltrials.gov NCT 03733990) indicates that patients that see a clinical benefit (tumor shrinkage or stable disease) from anti-CLEVER-1 therapy comprising an administration of anti-CLEVER-1 antibody bexmarilimab have at least 1 % of CLEVER-1 expressing intra-tumoral cells in a stained sample obtained prior to the beginning of the anti-CLEVER-1 therapy, calculated from the total amount of viable intra-tumoral cells present in the stained sample. According to performed ROC analyses a threshold of a CLEVER-1 intra-tumoral score for achieving benefit from anti-CLEVER-1 therapy (bexmarilimab) could be set at range of 3-7%. A range of an intra-tumoral CLEVER-1 positive score of 1 - 10% is an optimal according to an embodiment of the invention. An intra-tumoral CLEVER-1 positive score of 1% is required for benefitting anti-CLEVER-1 therapy.

The present invention comprising an administration of anti-CLEVER-1 antibody bexmarilimab is most valuable for patients having diagnosed with a tumour which comprises a substantial amount of CLEVER-1 positive intra-tumoral cells. According to the present invention, it has been observed that anti-CLEVER-1 antibody bexmarilimab is most valuable for cancer patient having diagnosed with a tumor which comprises at least 1% of intra-tumoral CLEVER-1 expressing cells from the total amount of the viable intra-tumoral cells in a sample obtained from the tumor. In an embodiment of the present invention, a tumor sample which comprises 1 - 10 % or 3 - 7 % of CLEVER-1 expressing intra-tumoral cells from the total amount of viable intra-tumoral cells, is an indication that the cancer patient is responsive to the anti-CLEVER-1 therapy.

Hence, according to an embodiment of the present invention intra-tumoral CLEVER-1 expression can be used as the predictive biomarker prior to the treatment for determining patient's responsiveness to anti-CLEVER-1 therapy. It has been observed that in CLEVER-1 staining only the amount of positive cells that are intra-tumoral are meaningful. In the method according to the present invention, the percentage of CLEVER-1 intra-tumoral expression level is calculated from the total amount of viable intra-tumoral cells.

In the method according to the present invention, a decision to start an anti-CLEVER-1 therapy is made based on the pre-treatment *in vitro* diagnosis. A tumour which comprises the specified CLEVER-1 expression is indicative that the patient is responsive to the anti-CLEVER-1 therapy. According to the present invention, anti-CLEVER-1 therapy or treatment refers to a treatment comprising an administration of anti-CLEVER-1 antibody, preferably anti-CLEVER-1 antibody bexmarilimab. In a typical method for treating a cancer patient, anti-CLEVER-1 antibody is administered therapeutically effective amount.

The term "treatment" or "treating" shall be understood to include complete curing of a disease or disorder, as well as amelioration or alleviation of said disease or disorder. The term "therapeutically effective amount" is meant to include any amount of an agent according to the present invention that is sufficient to bring about a desired therapeutic result. "Administering" refers to the physical introduction of a composition comprising said therapeutic agents to an individual, using any of the various methods and delivery systems known to those skilled in the art. The agents to be used in the present invention may be administered by any means that achieve their intended purpose. For example, administration may be intravenous, intramuscular, intraperitoneal, intra-tumoral, subcutaneous or other parenteral routes of administration, for example by injection. In addition to the pharmacologically active compounds, the pharmaceutical preparations of said agents preferably contain suitable pharmaceutically acceptable carriers comprising excipients and auxiliaries that facilitate processing of the active agents into preparations that can be used pharmaceutically. The dose chosen should be sufficient to reduce malignant tumor growth and/or inhibit metastasis formation.

The present invention for treating cancer by reducing malignant tumor growth and/or by inhibiting metastasis formation is applicable to all forms of cancers. Thus, any benign or malignant tumor or metastasis of malignant tumor can be treated.

### EXPERIMENTAL PART

### First-in-human clinical study of an anti-CLEVER-1 antibody

CLEVER-1 inhibiting agent, an anti-CLEVER-1 antibody FP-1305, is currently being tested for safety and preliminary efficacy in a Phase I/II study in patient with advanced solid tumors (clinicaltrials.gov NCT03733990: A Study to Evaluate Safety, Tolerability and Preliminary Efficacy of FP-1305 in Cancer Patients (MATINS)).

The anti-CLEVER-1 antibody FP-1305 is a humanized monoclonal CLEVER-1 antibody, previously presented in the patent publication WO2017/182705. More precisely, FP-1305 (DSM ACC3361) is a humanized monoclonal immunoglobulin G4κ antibody bexmarilimab (International Nonproprietary Name (INN)) as disclosed in WHO Drug Information, Vol. 34, No. 3 (2020), pages 699-700) produced in CHO cells.

In the present study, a pre-treatment tumour biopsy is obtained prior to starting anti-CLEVER-1 antibody FP-1305 treatment and the obtained tumor sample is fixated in formalin (FFPE) for later immunohistochemistry (IHC) staining and analysis by an independent centralized pathologist. Tumour progression or regression is evaluated repeating a CT scan that is compared to an existing scan taken before initiating anti-CLEVER-1 treatment. Progressive disease (PD) means the cancer is growing. No significant change in tumour size, which is a positive effect in aggressive otherwise non-treatable cancers, such as in the MATINS trial, is labelled as stable disease (SD), and considered good response. Tumour shrinkage is referred to as partial response (PR) according to the RECIST criteria used to evaluate treatment response. DCR (DCR = disease control rate) patients are patients who have achieved complete response, partial response and stable disease to a therapeutic intervention of anti-CLEVER-1 antibody FP-1305.

### IHC staining of tumor biopsies prior to anti-CLEVER-1 therapy

FFPE (formalin-fixed paraffin-embedded) tumor samples obtained from MATINS Trial patients of 4-5 µm sections were stained with Ventana Benchmark Ultra (Roche Diagnostics, Basel, Switzerland). For CLEVER-1 stainings, UltraView Universal DAB Detection Kit (Roche Diagnostics) combined with CLEVER-1 primary antibody (monoclonal IgG2a kappa STAB-1 antibody (clone 4G9), Abnova (Taiwan) Corporation) at 1:100 dilution was used. CLEVER-1 staining from DCR patients are shown in Figure 1. Interpretation and scoring were performed by board-certified pathologists using bright field microscopy. Percentage of CLEVER-1 positive viable cells (the number of all CLEVER-1 positive cells divided by the total number of cells, multiplied by 100) were scored irrespectively of location, and also percentage of CLEVER-1 positive viable cells were scored intratumorally and in stroma. Results are shown in Figure 2.

Figure 2 shows the percentage viable cells (VCs) that are positive for CLEVER-1 staining from the amount of total cells in a biopsy obtained from patient prior to anti-CLEVER-1 therapy in the MATINS Trial, and their association with clinical benefit (DCR patients). Also, a percentage of CLEVER-1 expressing cells in stromal and intra-tumoral viable cells are shown. It has been observed that DCR patients had higher intra-tumoral CLEVER-1 staining.

Based on the results, intra-tumoral CLEVER-1 expression levels were statistically significant to identify patients that benefit from anti-CLEVER-1 therapy from those that do not (p = 0.038) (Fig. 3). The percentage of expression levels is calculated from the total amount of viable cells present in the stained sample for intra-tumoral cells for CLEVER-1.

The data is further analyzed based on the Receiver operating characteristics (ROC) curve analysis from pre-treatment tumor biopsies from the first-in-human anti-CLEVER-1 trial named MATINS (clinicaltrials.gov NCT 03733990) investigating bexmarilimab (anti-CLEVER-1 mAb) for the treatment of advanced solid tumors. ROC curve is shown in Figure 4. The area under an ROC curve (AUC) indicates the accuracy of the method. In the current ROC analysis using pre-treatment intra-tumoral staining result, the AUC value was 0.72 (95% confidence interval from 0.51 to 0.92) indicating that CLEVER-1 immunohistochemical staining according to the present invention can separate patients that benefitted from anti-CLEVER-1 treatment. The ROC analysis consisted of 8 patients that benefitted from anti-CLEVER-1 treatment (tumor shrinkage or stabilization in follow-up imaging) compared to 75 patients that did not get benefit from anti-CLEVER-1 treatment (tumor progression in follow-up imaging).

### Cited references:

[1] Chen DS, Mellman I. Elements of cancer immunity and the cancer-immune set point. Nature 2017; 541(7637): 321-30.
[2] Virtakoivu et al. Systemic Blockade of Clever-1 Elicits Lymphocyte Activation Alongside Checkpoint Molecule Downregulation in Patients with Solid Tumors: Results from a Phase I/II Clinical Trial. Clin Cancer Res 2021; 27:4205-20.
[3] Guerriero JL, Sotayo A, Ponichtera HE, Castrillon JA, Pourzia AL, Schad S, et al. Class IIa HDAC inhibition reduces breast tumours and metastases through anti-tumour macrophages. Nature 2017; 543(7645):428-32.
[4] Qian BZ, Pollard JW. Macrophage diversity enhances tumor progression and metastasis. Cell 2010; 141(1):39-51.
[5] Kzhyshkowska J, Gratchev A, Goerdt S. Stabilin-1, a homeostatic scavenger receptor with multiple functions. J Cell Mol Med 2006;10(3):635-49.
[6] Kzhyshkowska J, Workman G, Cardó-Vila M, Arap W, Pasqualini R, Gratchev A, et al. Novel function of alternatively activated macrophages: stabilin-1-mediated clearance of SPARC. J Immunol 2006;176(10): 5825-32.
[7] Karikoski M, Marttila-Ichihara F, Elima K, Rantakari P, Hollmen M, Kelkka T, et al. Clever-1/Stabilin-1 Controls Cancer Growth and Metastasis. Clinical Cancer Research 2014;20(24):6452-64.
[8] WO 2010/122217 A1

## Claims

1. A method for pre-treatment identification of cancer patient that respond to anti-CLEVER-1 therapy comprising an administration of an agent capable of binding to Common Lymphatic Endothelial and Vascular Endothelial Receptor-1 (CLEVER-1) in a patient, **characterized in that** the method comprises
- detecting the presence of CLEVER-1 expressing cells in a tumor sample obtained from a cancer patient by immunohistochemistry staining using a mouse monoclonal IgG2a kappa STAB-1 antibody (clone 4G9), and
- calculating a percentage of intra-tumoral CLEVER-1 expressing cells from the total amount of viable intra-tumoral cells present in the stained tumor sample,
wherein the stained tumor sample which comprises at least 1 % of CLEVER-1 expressing intra-tumoral cells from the total amount of the viable intra-tumoral cells is an indication that the cancer patient is responsive to the anti-CLEVER-1 therapy.

2. The method according to claim 1, **characterized in that** the agent capable of binding to CLEVER-1 comprises anti-CLEVER-1 antibody, preferably anti-CLEVER-1 antibody bexmarilimab, bexmarilimab biosimilar or bexmarilimab variant.

3. The method according to claim 1 or 2, **characterized in that** the tumor sample is a tumor biopsy sample.

4. The method according to any one of the preceding claims, **characterized in that** the tumor sample is formalin fixed sample, formalin-fixed and paraffin-embedded (FFPE) sample or frozen sample.

5. The method according to any one of the preceding claims, **characterized in that** intra-tumoral cells comprise macrophages and/or tumor endothelial cells.

6. Use of a kit, comprising at least a mouse monoclonal IgG2a kappa STAB-1 antibody (clone 4G9) and reagents for staining a tumor sample, for carrying out a method of any one of claims 1 to 5.

## Patentansprüche

1. Verfahren zur Identifizierung von Krebspatienten vor der Behandlung, die auf eine Anti-CLEVER-1-Therapie ansprechen, umfassend die Verabreichung eines Mittels, das in der Lage ist, an den gemeinsamen lymphatischen endothelialen und vaskulären endothelialen Rezeptor-1 (CLEVER-1) zu binden, an einen Patienten, **dadurch gekennzeichnet, dass** das Verfahren Folgendes umfasst:
- Erkennen des Vorhandenseins von CLEVER-1 exprimierenden Zellen in einer von einem Krebspatienten erhaltenen Tumorprobe durch immunhistochemische Färbung unter Verwendung eines monoklonalen Maus-Antikörpers IgG2a kappa STAB-1 (Klon 4G9), und
- Berechnen des Prozentsatzes der intra-tumoralen CLEVER-1 exprimierenden Zellen aus der Gesamtmenge der lebensfähigen intra-tumoralen Zellen in der gefärbten Tumorprobe,
wobei die gefärbte Tumorprobe, die mindestens 1 % CLEVER-1 exprimierende intra-tumorale Zellen von der Gesamtmenge der lebensfähigen intra-tumoralen Zellen umfasst, eine Angabe darüber ist, dass der Krebspatient auf die antiCLEVER-1-Therapie anspricht.

2. Verfahren nach Anspruch 1, **dadurch gekennzeichnet, dass** das Mittel, das an CLEVER-1 binden kann, den Anti-CLEVER-1-Antikörper, vorzugsweise den Anti-CLEVER-1-Antikörper Bexmarilimab, das Bexmarilimab-Biosimilar oder die Bexmarilimab-Variante umfasst.

3. Verfahren nach Anspruch 1 oder 2, **dadurch gekennzeichnet, dass** die Tumorprobe eine Tumorbiopsieprobe ist.

4. Verfahren nach einem der vorstehenden Ansprüche, **dadurch gekennzeichnet, dass** die Tumorprobe eine in Formalin fixierte Probe, eine in Formalin fixierte und in Paraffin eingebettete (FFPE) Probe oder eine gefrorene Probe ist.

5. Verfahren nach einem der vorstehenden Ansprüche, **dadurch gekennzeichnet, dass** die intra-tumoralen Zellen Makrophagen und/oder Tumorendothelzellen umfassen.

6. Verwendung eines Kits, das mindestens einen monoklonalen Maus-Antikörper IgG2a kappa STAB-1 (Klon 4G9) und Reagenzien zum Färben einer Tumorprobe umfasst, zur Durchführung eines Verfahrens nach einem der Ansprüche 1 bis 5.

## Revendications

1. Procédé d'identification de pré-traitement de patients atteints d'un cancer qui répondent à une thérapie anti-CLEVER-1 comprenant une administration d'un agent capable de se lier au récepteur endothélial lymphatique et endothélial vasculaire commun de type 1 (CLEVER-1) chez un patient, **caractérisé en ce que** le procédé comprend
- la détection de la présence de cellules exprimant CLEVER-1 dans un échantillon de tumeur obtenu à partir d'un patient atteint d'un cancer par coloration immunohistochimique à l'aide d'un anticorps lgG2a kappa STAB-1 monoclonal murin (clone 4G9), et
- le calcul d'un pourcentage de cellules intratumorales exprimant CLEVER-1 par rapport à la quantité totale de cellules intratumorales viables présentes dans l'échantillon de tumeur coloré,
dans lequel l'échantillon de tumeur coloré qui comprend au moins 1 % de cellules intratumorales exprimant CLEVER-1 par rapport à la quantité totale de cellules intratumorales viables est une indication selon laquelle le patient atteint d'un cancer est sensible à la thérapie anti-CLEVER-1.

2. Procédé selon la revendication 1, **caractérisé en ce que** l'agent capable de se lier à CLEVER-1 comprend un anticorps anti-CLEVER-1, de préférence un anticorps anti-CLEVER-1 bexmarilimab, un biosimilaire du bexmarilimab ou un variant du bexmarilimab.

3. Procédé selon la revendication 1 ou 2, **caractérisé en ce que** l'échantillon de tumeur est un échantillon de biopsie tumorale.

4. Procédé selon l'une quelconque des revendications précédentes, **caractérisé en ce que** l'échantillon de tumeur est un échantillon fixé au formol, un échantillon fixé au formol et inclus en paraffine (FFPE) ou un échantillon congelé.

5. Procédé selon l'une quelconque des revendications précédentes, **caractérisé en ce que** les cellules intratumorales comprennent des macrophages et/ou des cellules endothéliales tumorales.

6. Utilisation d'un kit, comprenant au moins un anticorps IgG2a kappa STAB-1 monoclonal murin (clone 4G9) et des réactifs pour colorer un échantillon de tumeur, pour mettre en œuvre un procédé selon l'une quelconque des revendications 1 à 5.
